# EUROPEAN PATENT SPECIFICATION

(11) **EP 1 871 422 B1**
(45) Date of publication and mention of the grant of the patent: **16.10.2013**
(21) Application number: 06733314.6
(22) Date of filing: 18.04.2006
(51) Int. Cl.: A61K 47/48, A61P 35/00, B01J 23/42, B01J 35/00, B22F 1/00, G01N 33/49

(54) **USE OF RHCC PEPTIDE IN DRUG DELIVERY**
VERWENDUNG VON RHCC-PEPTID BEI DER ARZNEIABGABE
UTILISATION DU PEPTIDE RHCC DANS L'ADMINISTRATION D'UN MEDICAMENT

(30) Priority: 18.04.2005 US 672403 P
(43) Date of publication of application: 02.01.2008
(73) Proprietor: Archaea Pharma AB, 114 46 Stockholm (SE)
(72) Inventor: MUELLER, Juergen, 791 89 Bad Krozingen (DE); FIGGEMEIER, Egbert, 51371 Leverkusen (DE)
(74) Representative: Stenbäck, Maria Elisabeth
(86) International application number: PCT/SE2006/000458
(87) International publication number: WO 2006/112777

(56) References cited:
- STETEFELD J ET AL: "CRYSTAL STRUCTURE OF A NATURALLY OCCURRING PARALLEL RIGHT-HANDED COILED COIL TETRAMER" NATURE STRUCTURAL BIOLOGY, NEW YORK, NY, US, vol. 7, no. 9, 2000, pages 772-776, XP008069372 ISSN: 1072-8368
- ÖZBEK, S. ET AL: "Favourable mediation of crystal contacts by cocoamidopropylbetaine (CAPB)" ACTA CRYSTALLOGRAPHICA SECTION D, vol. D61, 2005, pages 477-480, XP008073022
- DURE L.: "A repeating 11-mer amino acid motif and plant desiccation" THE PLANT JOURNAL, vol. 3, no. 3, 1993, pages 363-369, XP008073056
- PETERS J ET AL: "HYPERTHERMOSTABLE SURFACE LAYER PROTEIN TETRABRACHION FROM THE ARCHAEBACTERIUM STAPHYLOTHERMUS MARINUS: EVIDENCE FOR THE PRESENCE OF A RIGHT-HANDED COILED COIL DERIVED FROM THE PRIMARY STRUCTURE" JOURNAL OF MOLECULAR BIOLOGY, LONDON, GB, vol. 257, no. 5, 1996, pages 1031-1041, XP008069366 ISSN: 0022-2836
- WOOLFSON D. N.: "The design of coiled-coil structures and assemblies" ADVANCES IN PROTEIN CHEMISTRY, vol. 70, 2005, pages 79-112, XP008073019
- DEL RIZZO P. A. ET AL: "ATP synthase b subunit dimerization domain: a right handel coiled coil with offset helices" J. MOL. BIOL., vol. 364, 2006, pages 735-746, XP008073020
- PARRY ET AL: "Hendecad repeat in segment 2A and linker L2 of intermediate filament chains implies the possibility of a right-handed coiled-coil structure" JOURNAL OF STRUCTURAL BIOLOGY, ORLANDO, US, vol. 155, no. 2, August 2006 (2006-08), pages 370-374, XP005611219 ISSN: 1047-8477

## Description

### Field of the invention

The present invention relates to the field of proteins and their use in drug delivery . More specifically, the invention relates to the use of a specific protein comprising hydrophobic cavities suitable for use in drug delivery .

### Background of the invention

The polypeptide chain called RHCC (right-handed coiled-coil) is a fragment of the tetrabrachion protein, which is produced by *Staphylothermus marinus -* a bacterium living in the environment of so-called "black smokers" on the sea ground. The bacterium is sulphur dependent and has an optimal growth temperature of 92 °C and lives on the fermentation of peptides. The bacterium has a quasi-periplasmic space containing so-called stacks, which are built of four-stranded helical coiled-coils together with two proteases". A remarkable property of the bacterium, tetrabrachion and RHCC is the extreme thermostability and its strong resistance against denaturants.

The structure of RHCC has been established by x-ray crystallography. It is a tetrameric assembly with the fragments being right-handed helices and a right-handled superstructure. Each of the four RHCC strands contains 52 residues and comprises the protease-binding region of tetrabrachion (amino acid residues 1238-1287). RHCC is on average 72 A long and has a diameter of 25 A. While the outside of the rod-like protein is rather hydrophilic, the inside has strongly hydrophobic character and a large buried surface of roughly 9500 A². Most characteristic is a channel through the entire tetramer, which is accompanied by 4 large cavities. The cavities have volumes ranging from 145 to 300 A³, and also strongly hydrophobic character.^{ii,iii,iv,v} Nevertheless, in the crystal structure, these cavities are filled with water molecules.

Metals and metal ions and metal complexes have been applied for medical purposes long before their modes of action were known. An example of this is bismuth, used for treating gastrointestinal disorders. Modes of actions are being investigated, and metallodrug interactions with cell membranes, protein, DNA and enzymes are pointed at as being especially important. Understanding modes of actions makes design of metal-based drugs possible. Examples of areas where metallodrugs have made progress are silver based anti-infective agents, gadolinium and iron based contrast agents for magnetic resonance imaging, radionuclides for x-ray imaging, and platinum, e.g. cisplatin, for cancer treatment.^{vii}

However, as well as being powerful as therapeutic agents, metals can be very toxic when ingested uncontrolled. Heavy metals, for example lead, mercury, arsenic, and many of their compounds are toxic to humans, yet the exposure to these and others have increased by the industrialisation of society. The toxic effect may be the result of complex formation between the metal compound and the natural constituents of the body, for example oxygen, sulphur and nitrogen. The biological molecules modified by the metals may loose their abilities to function properly and signs of this may be pathologic if the metal is ingested in sufficient quantities.

### Drug delivery

Most drugs (e.g. cisplatin) have serious side effects, which mean that they are not only active against e.g. bacteria, viruses and cancer cells, but that they also attack body cells and organs. In order to minimize these side effects it is desirable to have a container, which screens the drug when moving through the body until it has reached the target cells or organs.

Many drugs are not soluble to the desired extent in the blood stream, so only small concentrations in the blood can be applied. In order to overcome this problem drug delivery systems (DDS) have been developed, which provide a hydrophilic shell around a hydrophobic core, which adapts the hydrophobic drug (e.g. liposomes).

The properties of drugs - metal-containing and non-metal-containing - can be improved by applying the drug in combination with a drug delivery system (DDS). DDS may change the pharmacokinetics and biodistribution (e.g. targeted delivery) of a given drug. DDS may be used as a drug reservoir for controlled release of a drug over a long period of time," Lipids and polymers are commonly used as DDS, and a widespread research has developed around these delivery systems. Lately also dendrimers and dendritic polymers have appeared on the market for the controlled delivery of drugs and/or contrast agents for imaging purposes.^{viii} Lately, a number of protein based DDS have emerged including protein-polymer systems as well as coiled-coil proteins.^{ix,x,xi}

DDS known in the field today still suffer serious drawbacks, as listed below. A typical DDS should be readily soluble in the blood stream so that sufficient amounts of the carrier-drug complex can be applied to the patient. Unfortunately, polymers and dendrimers used for DDS can have solubility problems, if the polymer does contain hydrophobic side chains or the dendrimer has a hydrophobic outer shell.

Specific targeting is desired when the drug is to be released from the DDS at a certain location in the body. Targeting could be achieved by adding a tag to the DDS, the tag being specific to the target tissue or a component on the target tissue. The most common delivery systems are difficult to tag; liposomes are difficult to tag because they consist of hundreds of single lipid molecules (lipids), which are aggregated. For liposome-based DDS, adding a tag to the lipid molecules may change the concentrations and procedures necessary to obtain liposomes. Also, the lipid tagging procedure may have to be performed in organic solvents, which may not be compatible with desired tags, which may be for example peptide-based structure (low solubility and stability in organic solvents). The same fundamental problems occur with polymers and dendrimers.

The DDS should not diminish unacceptably the functionality of the drug. For e.g. cancer treatment only the well-defined shape of the hydrolyzed form of cisplatin is able to bind to the RNA of the cancer cell and prevent its multiplication. There are attempts to deliver e.g. cisplatin to cancer cells by tagging it with small receptor-targeting molecules, but this is very challenging under the requirement to maintain the geometrical and chemical shape and thereby the functionality, of the cisplatin^{xiii}.

The DDS should be possible to produce in large quantities in a reproducible way. The production of liposomes is difficult to upscale; very often the properties of liposomes produced in large quantities do not match the ones produced in small quantities.

DDS based on polymers depends on a narrow molecular mass distribution of the polymer. This requires rather difficult production techniques and/or separation steps. Dendrimers are very often difficult to produce since its production is usually a multi-step chemical synthesis.

A useful DDS should be stable and possible to store. Liposomes are only metastable and degrade at room temperature, thus putting restrictions on post-production handling of the DDS.

### Metal filtering

Treatment of metal poisoning is focused on withdrawal of the metal from the body. A commonly used treatment is ethylenediamine-tetra-acetic acid (EDTA), a metal chelator complexing with the positively charged metal ions. EDTA does also complex with cations responsible for the integrity of cell membranes, like for example calcium and magnesium, and side-effects and immune-reactions are not ignorable.

### Nanoparticle production

Nano-sized materials (e.g. metal nano clusters) have attracted a massive interest over the last two decades. The interest is stirred by the prospect of an infinite number of properties and applications, which are connected to the effects of controlled ordering of atoms and .small molecules to nano-sized objects. The applications reach from new electronic devices based on quantum dots, sensors and catalysts to new classes of materials with unmatched mechanical strength (e.g. carbon nanotubes). A particular feature of metal nanoparticles is that they behave like molecules rather than bulk metals; e.g. such small particles have distinct absorption bands due to distinct energy levels in contrast to the continuous band structure of bulk metals. This effect can be exploited for determination of the size of metal nanoparticles.

To produce nanoparticles, a large number of procedures have been published. A particular challenge for the production of nanoparticles is to achieve a narrow size distribution^{xii}. A major problem when constructing nanoparticles is the aggregation of them to bigger units. Thiols have been used to solve this problem, as they bind to e.g. gold particle surfaces, preventing particle-particle interactions.

### Summary of the Invention

The present invention relates to a peptide derived from the tetrabrachion protein, herein named RHCC, and/or a fragment thereof as drug delivery system. Said RHCC peptide is a four-stranded coiled coil having cavities in which e.g. metal-complexes may accumulate.

The present invention provides new uses for said RHCC peptide and/or a fragment thereof in a single and/or in a crystallized form. Said RHCC peptide and/or crystal complex of peptides may be tagged at the individual peptides and/or on the crystal circumference, allowing for specific targeting of an environment and/or site in e.g. a living body, or wherever an effect is desirable. In one aspect, such a target may also be a micro organism e.g. bacterium and/or a virus. It will be understood by the person skilled in the art, that at least one RHCC peptide and/or a crystal complex of at least two RHCC peptides and/or fragments of peptides is intended, whenever an RHCC peptide and/or a fragment thereof is referred to.

The present invention relates to said RHCC peptide and/or a fragment thereof for use as a drug delivery system for the delivery of a drug comprising at least one element, such as a metal element, for association of said drug to said drug delivery system, to a specific site in the body. Said drug delivery system and a drug of choice is encompassed in a pharmaceutical composition, for use as a medicament.

### Brief description of the drawings

Figure 1: Absorption spectra of Hgl₄²⁻ dissolved in tris-buffer (**A**), in a mixture (vol. 50/60) of methanol and tris-buffer (**B**), in methanol (**C**) and in a tris-buffer solution together with RHCC (**E**). The spectrum of the tris-buffer (**D**) has been subtracted from all spectra and the spectrum of RHCC has been subtracted from spectrum **E**.
Figure 2: Absorption spectra of Hgl₄²⁻ dissolved in tris-buffer (A) in an unfiltered mixture with RHCC (B) and after filtering with size exclusion filters ("Centurion 10") (C).
Figure 3: Absorption spectra of cisplatin and RHCC (inset) in separate solutions and absorption spectra of mixed solution of RHCC and cisplatin after filtering excess cisplatin and replacement of solvent by metal complex free solvent.

### Detailed description of the invention

Surprisingly, the inventors have found that the possible binding, as well as release of a substance and/or a drug, to/from an RHCC peptide and/or a fragment thereof, renders it a valuable tool in drug delivery

Consequently, the main purpose of the present invention is to provide a system for drug delivery using an RHCC peptide and/or a fragment thereof, which will make it possible to both associate and release a substance and/or a drug at a specific site of interest.

Accordingly, the present invention relates to various uses of an RHCC peptide and/or a fragment thereof. The present inventors show that an RHCC peptide is able to associate to a substance and/or a drug of interest, which comprises an element enabling association to said RHCC peptide, said substance and/or drug associating to the interior of the peptide, subsequently being released by changed conditions in a target environment. Said RHCC peptide and/or fragment thereof may thus be used as a drug delivery system . Furthermore, said RHCC peptide and/or fragment thereof may also be comprised in a pharmaceutical composition further encompassing a drug and/or a substance of choice for the delivery of said drug and/or substance to e.g. a target tissue.

An RHCC peptide and/or a fragment thereof may in one context of the present invention be fused with a suitable tag (e.g. a single chain antibody recognizing cell surface antigens on cancer cells) e.g. by cloning the according cDNA fragment coding for the tag into the pet15b-RHCC construct by using restriction sites that will safeguard an insertion adjacent to the RHCC sequence. Expression and/or purification of the fusion peptide may be accomplished according to the expression and purification of RHCC alone as described in the experimental section. It is to be understood by the person skilled in the art that said RHCC peptide may of course also be fused with a tag after production and purification of the RHCC peptide.

**Table 1: Amino acid sequence of one strand of RHCC (SEQ.ID.NO:1)**

| | **a** | **b** | **c** | **d** | **e** | **f** | **g** | **h** | **i** | **j** | **k** |
|---|---|---|---|---|---|---|---|---|---|---|---|
| **1-3** | | | | | | | | | **G** | **S** | **I** |
| **4-11** | **I** | **N** | **E** | **T** | **A** | **D** | **D** | **I** | | | |
| **12-18** | | | | | **V** | **Y** | **R** | **L** | **T** | **V** | **I** |
| **19-29** | **I** | **D** | **D** | **R** | **Y** | **E** | **S** | **L** | **K** | **N** | **L** |
| **30-40** | **I** | **T** | **L** | **R** | **A** | **D** | **R** | **L** | **E** | **N** | **I** |
| **41-51** | **I** | **N** | **D** | **N** | **V** | **S** | **T** | **I** | **L** | **A** | **S** |
| **52** | **I** | | | | | | | | | | |

An RHCC peptide in the present context comprises a peptide comprising four strands, called peptide strands, together forming a right-handed parallel RHCC tetramer. A sequence of one of said peptide strands is given in Table 1 (SEQ.ID.NO:1), A tetrameric RHCC peptide comprises four peptide strands of 52 amino acid residues each, in total 208 amino acids. 11-residue repeat positions, indicated by lowercase letters, are assigned according to the model proposed by Lupas^{XIII} and Stetefeld^{II}. Hydrophobic core positions are a and h. The continuity of the 11-residue repeats is interrupted by a four-residue insertion (stutter) between Ile 11 and Thr 16. The first two N-terminal residues, Gly 1 and Ser 2 are not part of the tetrabrachion coding sequence. The native RHCC starts with II. The GS originates from the vector and does not belong to the Tetrabrachion protein. As we have performed all experiments with the recombinantly expressed protein starting with GS we referred to this sequence. In the fusion molecule these amino acids function as a linker. Numbering of the amino acids is indicated on the left of the sequence. Amino acid residue Ile 3 corresponds to position 1238 on the tetrabrachion sequence. Accordingly, in the present context, the term "RHCC peptide" refers to a tetrameric complex comprising four peptide strands, and/or to a fragment thereof, which peptide strands, independently of each other, may be of any suitable length, such as about 1 to 52 amino acids, derived from SEQ ID NO: 1. In one preferred embodiment of the present invention, a peptide strand is about 52 amino acids. An "RHCC peptide" may also refer to an RHCC peptide and/or to a fragment thereof, which has been modified in any suitable manner, such as by a point mutation, as disclosed by the present invention. It is furthermore to be understood that an "RHCC peptide" may also refer to a crystal complex of several RHCC peptides and/or fragments thereof. Such a crystal complex comprises at least two RHCC peptides and/or fragments thereof, with a preferred size of 10 nm³ and above. Such an RHCC crystal complex may additionally be of any suitable size for the aimed purpose of use.

Proteins are biological macromolecules constituted by amino acid residues linked together by peptide bonds. In the present context, proteins and/or peptides may be held together by interactions such as by hydrophobic and/or hydrophilic interactions and/or salt bridges. Proteins, as linear polymers of amino acid residues, are also called polypeptides. Typically, proteins have 50-800 amino acid residues, and hence have molecular weights in the range of from about 6,000 to several hundred thousand Dalton or more. Small proteins are called peptides or oligopeptides. However, in the present context, it is to be understood that the term "protein" may be used interchangeably with the terms "peptide" and "polypeptide".

Proteins, polypeptides, peptides, such as an RHCC peptide and/or fragments thereof, related to in this invention may be in a substantially isolated and/or purified form. It will be understood that the proteins, polypeptides and/or peptides may be mixed with carriers or diluents, which will not interfere with the intended purpose of the proteins, polypeptides and/or peptides and they will still be regarded as substantially isolated. Such a substantially purified form will generally comprise the proteins, polypeptides, and/or peptides in a preparation and/or a composition in which more than approximately 90%, e.g. 95%, 96%, 97%, 98%,99% or 100% of the proteins, polypeptides, and/or peptides in the preparation is a protein, polypeptide, and/or peptide, according to the invention.

Furthermore, any amino acid sequence being at least 70% identical, such as being at least 72%, 75%, 77%, 80%, 82%, 85%, 87%, 90%, 91%, 92%, 93%, 94%, 95%, 96%, 97%, 98%, or 99% identical with the amino acid sequence of a RHCC peptide and/or a fragment thereof according to the invention, is also considered to be inside the scope of the present invention.

A "fragment" according to the invention, comprises any suitable amino acid fragment of any length of an RHCC peptide. Accordingly, a fragment of an RHCC peptide may in the context of the present invention comprise at least one part of one peptide strand of a tetrameric RHCC peptide, up to at least one part of the four strands of an RHCC peptide. An RHCC peptide fragment according to the invention, comprises about 1-207 amino acids, such as, but not limited to, about 1 to 50, 50 to 100, 100 to 150, 150 to 200, or 200 to 207 amino acids. A fragment of an RHCC peptide may also be longer if any of said RHCC peptide strands has been extended with additional amino acid residues, which are not part of the natural amino acid sequence of an RHCC peptide strand (SEQ ID NO:1). The amount of additional amino acid residues to be added to the natural sequence is not limited, it is however envisaged that a binding domain may encompass about 100-150 residues.

An RHCC peptide and/or a fragment thereof can be produced in a number of different ways, including, fragmentation of larger molecules, chemical synthesis, recombinant technology, and/or by a combination of these methods. It is to be understood by the person skilled in the art, that any method for producing an RHCC peptide and/or a fragment thereof may be used in the context of the present invention.

Encompassed by the present invention is also the use of an RHCC peptide and/or a fragment thereof, which has been modified in any suitable manner. By a protein, polypeptide, peptide and/or a fragment thereof having an amino acid sequence at least, for example, 90% identical to a reference amino acid sequence, is intended that the amino acid sequence of e.g. the peptide is identical to the reference sequence, except that the amino acid sequence may include up to 10 point mutations per each 100 amino acids of a reference amino acid sequence. In other words, to obtain a peptide having an amino acid sequence e.g. at least 90% identical to a reference amino acid sequence up to 10% of the amino acids in the reference sequence may be deleted or substituted with another amino acid, or a number of amino acids. These mutations of the reference sequence may occur at the amino or carboxy terminal positions of the reference amino acid sequence or anywhere between those terminal positions, interspersed either individually among amino acids in the reference sequence or in one or more contiguous groups within the reference sequence. One or more point mutations in the sequence of one or more of the four individual strands of an RHCC peptide and/or a fragment thereof, are designed to improve the functionality of the RHCC peptide such as the binding and release of a substance, such as a metal containing substance, and/or a drug. Consequently, one context of the present invention relates to the use of an RHCC protein and/or a fragment thereof, which has been point mutated at any suitable position in a RHCC peptide and/or fragment thereof, to obtain an improved functionality. Any suitable amount of point mutations may be used to obtain an RHCC peptide and/or a fragment thereof with desired functionality. In one aspect of the invention, a point mutation is performed in at least one peptide strand of an RHCC peptide and/or a fragment thereof, to change a property of at least one cavity of said RHCC peptide.

Modifications of the RHCC peptide and/or a fragment thereof further include the addition of a tag, i.e. the addition of an amino acid, carbohydrate and/or a chemical substance, to one or more of the RHCC peptides and/or fragments thereof and/or to a RHCC peptide strand, achieved through joining the coding sequence of RHCC and any other peptide or protein within an expression vector when producing a RHCC peptide and/or fragment thereof in a living organism, or achieved through the binding by chemical means to one or more of the peptide strands of a RHCC peptide during or after the synthesis. Such a tag may be associated to a single RHCC peptide and/or to a fragment thereof and/or to a complex of RHCC peptides and/or fragments thereof of at least two RHCC peptides.

Additionally, the present invention of course also comprises the use of any other variant and/or derivative of an RHCC and/or a fragment thereof, that is still able to bind a desired substance and/or drug and/or which is still functionally equivalent to a natural RHCC peptide and/or a fragment thereof. Such a variant may be an RHCC peptide and/or fragment thereof, which has been extended by adding any suitable amount of amino acids to said RHCC peptide and/or fragment thereof, such as between 1-5 and 5-10 amino acids. Modifications of an RHCC peptide and/or fragment thereof according to the present invention, furthermore includes truncations. It should be understood that any modifications as disclosed by the present invention, may be performed in the nucleic acid and/or amino acid sequence of one or more of the RHCC peptide strands and/or fragments thereof.

In the present invention, a local algorithm program is best suited to determine identity. Local algorithm programs, (such as Smith-Waterman) compare a subsequence in one sequence with a subsequence in a second sequence, and find the combination of subsequences and the alignment of those subsequences, which yields the highest overall similarity score. Internal gaps, if allowed, are penalized. Local algorithms work well for comparing two multidomain proteins, which have a single domain or just a binding site in common.

Methods to determine identity and similarity are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. et al (1990)). The BLASTX program is publicly available from NCBI and other sources (BLAST Manual, Altschul, S.F. et al, Altschul, S.F. et al (1990)). Each sequence analysis program has a default scoring matrix and default gap penalties. In general, a molecular biologist would be expected to use the default settings established by the software program used.

An RHCC peptide and/or a fragment thereof may further in one context of the invention be applied as singular peptides and/or as crystal complexes of two or more RHCC peptides and/or fragments thereof, whereby appropriate sizes of said crystal may be designed. Furthermore, a RHCC peptide, in singular or crystal structure, may be modified with a tag with the purpose of accumulating the RHCC peptide at a site of interest. Typically, this site may be a tumour, and the tag may be a molecule specifically binding to the tumour-type in question. The site to which the tag is specific, being a tissue or a constituent of a tissue, is called the target, target tissue or targeted tissue. Such a target may also in one aspect of the present invention, comprise a micro organism such as a bacterium and/or a virus.

### RHCC as a drug delivery system (DDS)

In a preferred embodiment, the present invention relates to the use of an RHCC peptide and/or a fragment thereof as a drug delivery system for administering a drug comprising at least one element for association of said drug to said drug delivery system. In one aspect, said element is a metal element.

It is a particular objective of the present invention to provide a new DDS comprising an RHCC protein, and/or a fragment thereof, which reduces the unintentional toxicity of drugs, such as metal containing drugs.

The term "drug delivery system" in the present context refers to a system for the delivery of a drug of any kind, such as disclosed herein, which comprises an RHCC peptide and/or a fragment thereof, which is able to associate said drug of interest into a cavity of said RHCC peptide, to carry said drug in a body, and to subsequently release said drug from said cavity by diffusion or following or brought about by a change of conditions in a target environment when this is reached.

In the context of the present invention, the term "element" refers to any matter which is a part of a substance and/or a drug to be associated into a cavity of a RHCC peptide and/or fragment thereof, and which is selected upon the basis of its ability to contribute to the association to said RHCC peptide and/or fragment thereof. Such an element may be, a metal element, a hydrophobic element, a hydrophilic element etc.

In the present context, the term "association" is used to describe the action of a drug and/or substance, which enters into a cavity of an RHCC peptide and/or fragment thereof subsequently associating thereto, wherein said association to an RHCC peptide may be performed by any interaction between the substance and/or drug in question with an RHCC peptide and/or fragment thereof, such as a hydrophobic interaction, a hydrophilic interaction, an electrostatic interaction, a covalent binding, a Van der Waals binding, an ion binding as well as combinations thereof.

In the context of the present invention, the term "drug" refers to a substance, and/or to a combination of substances, added to the body for a medical purpose. Such a medical purpose may be diagnosis, therapy, and/or imaging. A "drug" and/or a "substance" according to the invention relates to any chemical substance, peptide/protein, synthetic substance, etc, which is suitable for the intended purpose.

In the present context, "drug" and "substance" may be used interchangeably. In one embodiment of the present invention, metal-containing drugs are preferred. Such a drug and/or combination of drugs is administered in a suitable manner to a patient in a dose which will be designed for each patient depending on the patient's gender, age, heath status, illness etc.

Herein, unintentional toxicity refers to all non-wanted effects of the drug, including what is called side effects. A DDS which comprises an RHCC peptide and/or a fragment thereof may shield the body from a drug as the drug is kept within a peptide cavity. Without wishing to limit the invention, an RHCC peptide and/or a fragment thereof may furthermore shield the drug from degradation by bodily constituents.

In the present context, the term "metal element" refers to an element which comprises a metal constituent of any kind and/or amount, such as atoms of metals and/or half metals, as well as any applicable substance containing such, whether in charged or non-charged, hydrophobic, hydrophilic or any other form.

The element is selected from the group consisting of: Al, Sc, Ti, V, Cr, Fe, Co, Ni, Cu, Zn, Ga, Ge, As, Se, Sr, Y, Zr, Nb, Mo, Tc, Ru, Rh, Pd, Ag, Cd, In, Sn, Sb, Te, Cs, Ba, La, Ce, Pr, Nd, Pm, Sm, Eu, Gd, Tb, Dy, Ho, Er, Tm, Yb, Lu, Hf, Ta, W, Re, Os, lr, Pt, Au, Hg, TI, Pb, Bi, Fr, Ra, Ac, Th, Pa, Np, and Am, and/or a combination thereof. Furthermore, it is envisaged that said element may be a part of a substance and/or drug.

In yet another embodiment, said drug is selected from the group of Pt-containing drugs consisting of cisplatin (PtH₆N₂Cl₂), Carboplatin (PtC₆H₁₂N₂O₄), Nedaplatin (PtN₂C₂H₈N₂O₃), Oxaliplatin (PtN₂C₈H₁₄O₄), ZD0473 (PtC₆H₁₀N₂Cl₂), JM216 (PtC₁₀H₂₂N₂O₄Cl₂), Lobaplatin (PtC₉H₁₈N₂O₃), *trans*-[PtCl₂(pyridine)₂], and *trans-*[Pt(OH)₂Cl₂(NH₃)(NH₂-C₆H₅)], and/or a combination thereof.

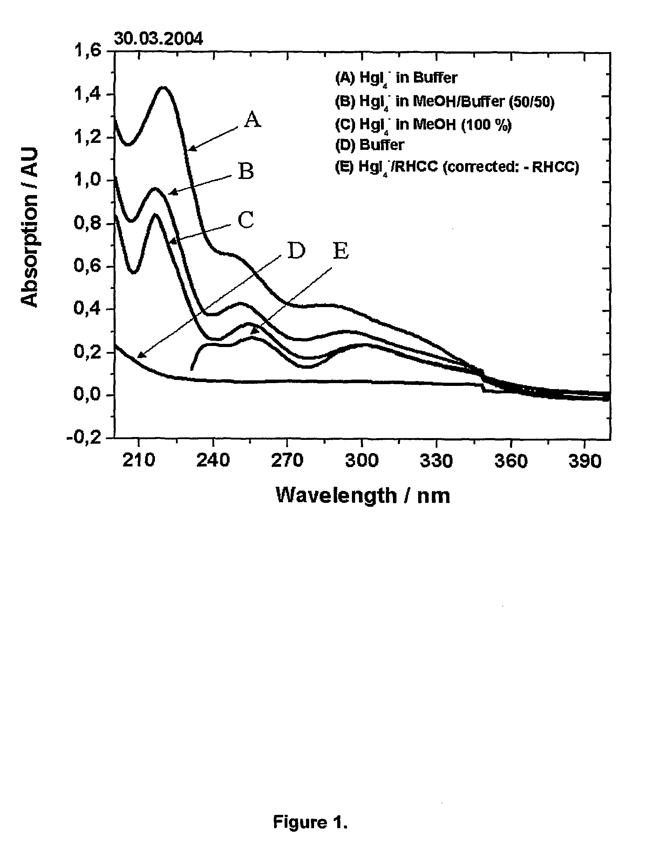

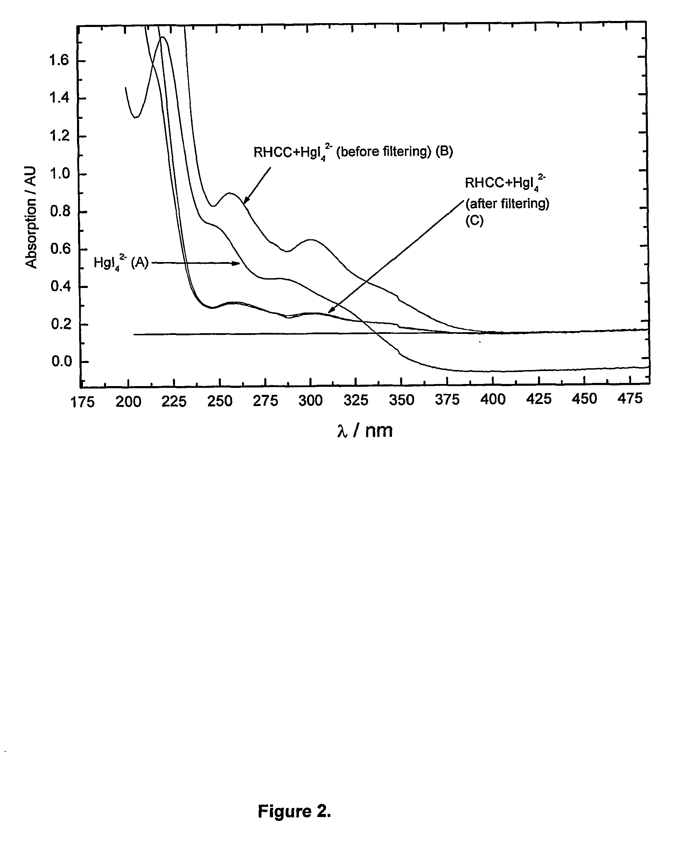

Said Pt-containing drugs and/or any other drugs for use in the context of the present invention are envisioned to be loaded into the cavities of an RHCC peptide and/or a fragment thereof. Without wishing to limit the scope of the present invention, the loading may be achieved through e.g. soaking of an RHCC peptide and/or an RHCC peptide crystal in a solution containing the drug, for example by mixing a 1 mM solution of cis-PtCl₂(NH₂)₂ with a 0.2 mM solution of the peptide. In one context of the present invention, the mixed solution is stirred for 1 hour at room temperature. It is however envisaged that mixing conditions as well as the temperature during such a mixing may be varied in any appropriate manner. The stability of an RHCC peptide and/or a fragment thereof allows loading at higher or lower temperatures depending on the drug properties. Examples of loading of drugs are given in Figure 1 and 2.

Furthermore, in one embodiment, the invention relates to use of an RHCC peptide and/or a fragment thereof as a drug delivery system for administering a drug comprising at least one element for association of said drug to said drug delivery system, as disclosed by the invention, wherein said RHCC peptide and/or fragment thereof further comprises a tag for specific targeting.

In the present context, the term "tag" refers to a molecular label which allows for selective targeting of a specific environment. A tag, according to the invention, interacts specifically with a target. In comparison to other DDS, i.e. liposomes, polymers and dendrimers, a tagged peptide can be produced by gene expression in bacteria. Furthermore, tagging of each peptide strand (e.g. with antibodies directed against cell surface antigens of cancer cells) may provide oligomerization of the tag (tetramer formation), thereby increasing the binding activity and biological activity (ref: Proc Natl Acad Sci USA 2004 April 13;101 (15):5547-52). Examples of a tag which may be associated to an RHCC peptide and/or a fragment thereof is an amino acid, carbohydrate and/or another chemical substance, which is added to one or more of the RHCC peptides and/or fragments thereof and/or to an RHCC peptide strand, achieved through adding a codon sequence to the coding vector when producing an RHCC peptide in a living organism, or achieved through the binding by chemical means to one or more of the RHCC peptide strands of the RHCC peptide during or after the synthesis. Such a tag may be associated to a single RHCC peptide, and/or to fragment thereof, and/or to a complex of RHCC peptides and/or fragments thereof, of at least two RHCC peptides.

When an RHCC peptide and/or fragment thereof reach the target, the drug may in one particular embodiment be released from the peptide. In the present context, the term "drug-release" refers to when a drug is dissociated from a cavity and/or a space to where it is associated to in an RHCC peptide and/or a fragment thereof. Such a dissociation may be due to diffusion and/or changes in an environment, such as a change in pH, enzymatic degradation of the RHCC peptide, hydrolytic degradation of the RHCC peptide, breakage of salt bridges opening the peptide and allowing leakage, or by the tag triggering RHCC peptide internalisation into the cell when interacting with the target etc. Particularly, leakage of the drug from the peptide will take place in the proximity of the intake systems of cells where the pH is lower as compared to the surrounding, the lower pH breaking the salt bridges. In one aspect of the invention, an RHCC peptide may be made to enter into the cell by means of the tag.

In another aspect, the invention also relates to the use of an RHCC peptide, and/or a fragment thereof, as a drug delivery system for administering a drug comprising at least one element for association of said drug to said drug delivery system, wherein a drug-release from said RHCC peptide and/or fragment thereof is due to a change of pH in a target environment, enzymatic degradation of an RHCC peptide and/or a fragment thereof, hydrolytic degradation of an RHCC peptide and/or fragment thereof, and/or opening of salt bridges of an RHCC peptide and/or a fragment thereof allowing leakage by a change in thermodynamic equilibria and/or kinetic barriers.

A "change of pH in a target environment" refers to a change in pH which is due to e.g. transport of an RHCC peptide and/or a fragment thereof, to another environment wherein the pH is higher or lower than in the original environment, resulting in changes, such as in the charge of said RHCC peptide and/or a fragment thereof, leading to release of a substance and/or drug present in a cavity of an RHCC peptide. In a preferred embodiment of the invention, pH is lower in a target environment, such as in a cancer cell, wherein said substance preferably is to be released for a specific effect.

A "target environment" and/or a "target" may in the context of the present invention be any tissue, cell type and/or fluid in a living body. Furthermore, in one context a target may also be a micro organism, such as a bacterium, or a virus, as well as any surface molecule present on such a micro organism or virus, It is to be understood that a "target environment" and/or a "target" for an RHCC peptide and/or a fragment thereof may be any site to where such an RHCC peptide is directed, e.g. by the use of a tag.

In one context of the present invention, a substance and/or a drug may also be facilitated by the immobilisation of an RCHH peptide and/or a fragment thereof at a desired target via specific binding, which can increase the probability of a substance and/or a drug being released at this site. Such a mode of release might especially be applied when an RHCC peptide and/or a fragment thereof is administered directly at the site of the target, being fixed to the target thereafter via specific binding to the target. A release of a substance and/or a drug is in this case mainly dependent on the binding equilibrium.

Without wishing to limit the scope of the present invention, it is envisaged that the drug will eventually be released from an RHCC peptide and/or a fragment thereof at the location of the desired action e.g. at the organ, infected cells and/or tumour cells, herein called the target and/or "target environment". As previously pointed out, an RHCC peptide and/or fragment thereof offers a number of alternative release mechanisms. A release may be triggered by e.g. biological (e.g. enzymatic degradation of the protein) and chemical processes.

Furthermore, it is preferred that the timing of the release, i.e. preferably very low or no release before reaching the target (organ, cells or tumours of concern) followed by a release at a desired rate at the target is controllable. Therefore the kinetic barrier of the drug transport between the inside of an RHCC peptide and/or a fragment thereof and the outside (solvent, intra-crystal space) is of importance.

The entrances of the cavities of an RHCC peptide are built by salt bridges and/or hydrogen bonds. Without the desire to limit the present invention, it is speculated that the plurality of salt bridges is a reason for the extreme stability of an RHCC peptide, and thus the reason why the drug will stay inside the peptide until release is triggered, and the salt bridges are opened.

The invention relates to a drug delivery system for administering a drug, wherein said drug delivery system comprises an RHCC peptide and/or a fragment thereof and a drug comprising at least one element selected from the group consisting of Al, Sc, Ti, V, Cr, Fe, Co, Ni, Cu, Zn, Ga, Ge, As, Se, Sr, Y, Zr, Nb, Mo, Tc, Ru, Rh, Pd, Ag, Cd, In, Sn, Sb, Te, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Fr, Ra, Ac, Th, Pa, Np, and Am, and/or a combination thereof. Furthermore, it is envisaged that said element may be a part of a substance and/or drug.

In another aspect, the invention relates to a drug delivery system wherein said drug is selected from the group of Pt-containing drugs consisting of cisplatin (PtH₆N₂Cl₂), Carboplatin (PtC₆H₁₂N₂O₄), Nedaplatin (PtN₂C₂H₈N₂O₃), Oxaliplatin (PtN₂C₈H₁₄O₄), ZD0473 (PtC₆H₁₀N₂Cl₂), JM216 (PtC₁₀H₂₂N₂O₄Cl₂), Lobaplatin (PtC₉H₁₈N₂O₃), *trans-*[PtCl₂(pyridine)₂], and *trans*-[Pt(OH)₂Cl₂(NH₃)(NH₂-C₆H₅)], and/or a combination thereof. Such a drug delivery system may also in one aspect of the invention comprise a tag, as disclosed by the present invention, for specific targeting.

The stability of an RHCC peptide also in harsh conditions and in a large temperature interval makes it superior to almost any other peptide (e.g. other coiled coils) thought of for drug delivery purposes. In comparison, the integrity of liposomes is difficult to control in the blood stream and therefore the drug-release from the liposome-based DDS is unpredictable.

An RHCC peptide is highly soluble in water because of its hydrophilic shell. A cavity of an RHCC peptide has a hydrophobic lining and drugs not so soluble in water, specifically metal-containing drugs, do accumulate in the cavities. The invention provides a DDS improving the solubility of poorly soluble drugs enabling the use of greater concentrations than otherwise. Furthermore, the peptide merely carries the drugs, implying that no chemical reactions between RHCC peptide and drug takes place, and the drug is delivered unchanged.

Preparation of an RHCC peptide and/or a fragment thereof, for drug delivery typically begins with mixing of an RHCC peptide and/or fragment thereof with a solution of the drug. Stirring may be necessary. The RHCC peptide is stable and temperature and other parameters may be set according to the properties of the selected drug.

A selected drug, e.g. cisplatin, may accumulate in a cavity of an RHCC peptide and/or a fragment thereof, creating a loaded peptide. In certain embodiments, said RHCC peptide is tagged to achieve accumulation at the target site. Association of the tag to the RHCC peptide is accomplished through any means known to the skilled in the area. For example, an RHCC peptide may be fused to a tag being an antibody designed to recognize antigens on target cells or tissues. The fusion may be facilitated by designing a gene, which incorporates both peptide and tag sequences, leading to the production of a fusion protein combining peptide and tag properties.

In one embodiment of the invention, an RHCC peptide and/or a fragment thereof loaded with a substance and/or a drug, with or without a tag or tags, is given to the patient i.v. (intravenously). If said drug is tagged, the RHCC peptide and/or a fragment thereof is by effect of the tag(s) accumulated at the targeted tissue.

Administration of a drug loaded into an RHCC peptide and/or a fragment thereof, alone or enscoped in a pharmaceutical composition, may be performed via any conventional administration route, such as an oral, parenteral, intravenous, buccal, aural, rectal, vaginal, intraperitoneal, topical (dermal), or nasal route, or by the administration to a body cavity. In one context, an RHCC peptide and/or a fragment thereof may be transported around in the body until hitting the target to which the tag is specific.

In another preferred embodiment, the invention relates to a pharmaceutical composition comprising a drug delivery system according to the invention.

A "pharmaceutical composition" refers to a composition which is suitable for a medical use, and which comprises a drug delivery system according to the invention, a drug and/or substance of choice, and optionally, an excipient. However, depending on the use of an RHCC peptide and/or a fragment thereof, a pharmaceutical composition may be a pharmaceutical and/or therapeutic composition.

A pharmaceutical composition comprising an RHCC peptide and/or a fragment thereof encompasses a drug delivery system in accordance with the invention. In the present context the term "drug delivery system" may be enscoped in a pharmaceutical composition (a pharmaceutical formulation or a dosage form) that upon administration presents the active substance and/or drug to the body of a human or an animal.

A pharmaceutically or cosmetically acceptable excipient is a substance that is substantially harmless to the individual to which the composition is to be administered. Such an excipient normally fulfils the requirements given by the national health authorities. Official pharmacopoeias such as e.g. the British Pharmacopoeia, the United States of America Pharmacopoeia and The European Pharmacopoeia set standards for pharmaceutically acceptable excipients.

The choice of pharmaceutically acceptable excipient(s) in a composition for use according to the invention and the optimum concentration thereof cannot generally be predicted and must be determined on the basis of an experimental evaluation of the final composition. A person skilled in the art of pharmaceutical and/or therapeutic formulation can find guidance in e.g., "Remington's Pharmaceutical Sciences", 18th Edition, Mack Publishing Company, Easton, 1990.

A pharmaceutical composition may be adapted to administration in connection with surgery, e.g. as a systemic administration by infusion into the blood, lymph, ascites, or spinal fluids, or by inhalation. For systemic application, a composition according to the invention may contain conventionally non-toxic pharmaceutically acceptable carriers and excipients according to the invention, including microspheres and liposomes.

A pharmaceutical composition for use in accordance with the present invention may be in the form of, e.g., a fluid, semi-solid or solid composition such as dissolved transfusion liquids, such as sterile saline, Ringer's solution, glucose solutions, phosphate buffer saline, blood, plasma, water, powders, microcapsules, bioabsorbable patches, drenches, sheets, bandages, plasters, implants, pills, sprays, soaps, suppositories, vagitories, toothpaste, lotions, mouthwash, shampoo, microspheres, nanoparticles, sprays, aerosols, inhalation devices, solutions, dispersions, wetting agents, suspensions, emulsions, pastes, ointments, hydrophilic ointments, creams, gels, hydrogels, dressings, devices, templates, smart gels, grafts, solutions, emulsions, suspensions, powders, films, foams, pads, sponges (e.g. collagen sponges), transdermal delivery systems, granules, granulates, capsules, agarose or chitosan beads, tablets, microcapsules, freeze-dried powders, granules, granulates or pellets, and mixtures thereof.

Suitable dispersing or wetting agents for use in accordance with the invention, may be naturally occurring phosphatides, e.g., lecithin, or soybean lecithin; condensation products of ethylene oxide with e.g. a fatty acid, a long chain aliphatic alcohol, or a partial ester derivable from fatty acids and a hexitol or a hexitol anhydride, e.g. polyoxyethylene stearate, polyoxyethylene sorbitol monooleate, polyoxyethylene sorbitan monooleate, etc.

Suitable suspending agents are, e.g., naturally occurring gums such as, e.g., gum acacia, xanthan gum, or gum tragacanth; celluloses such as, e.g., sodium carboxymethylcellulose, microcrystalline cellulose (e.g. Avicel® RC 591, methylcellulose); alginates and kitosans such as sodium alginate, etc.

Whether a pharmaceutically acceptable excipient is suitable for use in a pharmaceutical composition is generally dependent on which kind of dosage form is chosen for use for a type of disorder and/or damage to a body.

A pharmaceutically acceptable excipient may include solvents, buffering agents, preservatives, humectants, chelating agents, antioxidants, stabilizers, emulsifying agents, suspending agents, gel-forming agents, ointment bases, penetration enhancers, perfumes, powders and skin protective agents.

Examples of such solvents which may be used in a composition in accordance with the present invention, are water, alcohols, vegetable or marine oils (e.g. edible oils like almond oil, castor oil, cacao butter, coconut oil, corn oil, cottonseed oil, linseed oil, olive oil, palm oil, peanut oil, poppy seed oil, rape seed oil, sesame oil, soybean oil, sunflower oil, and tea seed oil), mineral oils, fatty oils, liquid paraffin, polyethylene glycols, propylene glycols, glycerol, liquid polyalkylsiloxanes, or other hydrophilic or etheric solvents such as weak acids with a pH of about 5,5-6,0 as well as mixtures thereof.

Examples of buffering agents are citric acid, acetic acid, tartaric acid, lactic acid, hydrogen phosphoric acid, bicarbonates, phosphates, diethylamine etc.

Suitable examples of preservatives are parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalconium chloride, and benzylalcohol, or mixtures of preservatives.

Examples of humectants are glycerin, propylene glycol, sorbitol, lactic acid, urea, and mixtures thereof.

Examples of chelating agents are sodium EDTA and citric acid.

Examples of antioxidants are butylated hydroxy anisole (BHA), ascorbic acid and derivatives thereof, tocopherol and derivatives thereof, cysteine, and mixtures thereof.

Examples of emulsifying agents are naturally occurring gums, e.g. gum acacia or gum tragacanth; naturally occurring phosphatides, e.g. soybean lecithin, sorbitan monooleate derivatives; wool fats; wool alcohols; sorbitan esters; monoglycerides; fatty alcohols; fatty acid esters (e.g. triglycerides of fatty acids); and mixtures thereof.

Examples of suspending agents are e.g. celluloses and cellulose derivatives such as, e.g., carboxymethyl cellulose, hydroxyethylcellulose, hydroxypropylcellulose, hydroxypropylmethylcellulose, microcrystalline cellulose, carraghenan, acacia gum, arabic gum, tragacanth, and mixtures thereof.

Examples of gel bases, viscosity-increasing agents or components which are able to take up exudate from a wound are: liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch or cellulose derivatives such as, e.g., carboxymethylcellulose, hydroxyethylcellulose and other cellulose derivatives, water-swellable hydrocolloids, carragenans, hyaluronates (e.g. hyaluronate gel optionally containing sodium chloride), collagen, gelatine, pectin, chitosans and alginates including propylene glycol aginate.

Examples of ointment bases are e. g. beeswax, paraffin, cetanol, cetyl palmitate, vegetable oils, sorbitan esters of fatty acids (Span), polyethylene glycols, and condensation products between sorbitan esters of fatty acids and ethylene oxide, e. g. polyoxyethylene sorbitan monooleate (Tween).

Examples of hydrophobic or water-emulsifying ointment bases are paraffins, vegetable oils, animal fats, synthetic glycerides, waxes, lanolin, and liquid polyalkylsiloxanes.

Examples of hydrophilic ointment bases are solid macrogols (polyethylene glycols).

Other examples of ointment bases are triethanolamine soaps, sulphated fatty alcohol and polysorbates.

Examples of powder components are: alginate, collagen, lactose, powder which is able to form a gel when applied to a wound (absorbs liquid/wound exudate). Normally, powders intended for application on large open wounds must be sterile and the particles present must be micronized.

Examples of other excipients are polymers such as carmelose, sodium carmelose, hydroxypropylmethylcellulose, hydroxyethylcellulose, hydroxypropylcellulose, methylcellulose, pectin, xanthan gum, locust bean gum, acacia gum, gelatin, carbomer, emulsifiers like vitamin E, glyceryl stearates, cetanyl glucoside, collagen, carrageenan, hyaluronates and alginates and kitosans.

Examples of diluents and disintegrating agents are lactose, saccharose, emdex, calcium phosphates, calcium carbonate, calcium sulphate, mannitol, starches and microcrystalline cellulose.

Examples of binding agents are saccharose, sorbitol, gum acacia, sodium alginate, gelatine, starches, cellulose, sodium coboxymethylcellulose, methylcellulose, hydroxypropylcellulose, polyvinylpyrrolidone and polyetyleneglycol.

It is appreciated that in those cases where a pharmaceutically acceptable excipient may be employed in different dosage forms or compositions, the application of a particular pharmaceutically acceptable excipient is not limited to a particular dosage form or of a particular function of the excipient.

Dressings and/or bandages are also important delivery systems for an RHCC peptide and/or a fragment thereof. When dressings are used as dosage form, an RHCC peptide and/or a fragment thereof may be admixed with the other material/ingredients before or during the manufacture of the dressing or, the peptide and/or the fragment thereof may in some way be coated onto the dressing e. g. by dipping the dressing in a solution or dispersion of the peptide and/or the fragment thereof, or by spraying a solution or dispersion of the fraction and/or peptide fragment onto the dressing. Alternatively, the peptide and/or fragment thereof may be applied in the form of a powder to the dressing. Dressings may be in the form of absorbent wound dressings for application to exuding wounds. Dressings may also be in the form of hydrogel dressings (e. g. cross-linked polymers such as, e.g. Intrasites which containscarboxymethylcellulose, propylene glycol or polysaccharide, disaccharide and proteins) or in the form of occlusive dressings such as, e. g., alginates, chitosan, hydrophilic polyurethane film, collagen sheets, plates, powders, foams, or sponges, foams (e. g. polyurethane or silicone), hydrocolloids (e. g.carboxymethylcellulose, CMC), collagen and hyaluronic acid-based dressings including combinations thereof.

In a pharmaceutical composition for use according to the invention, an RHCC peptide and/or a fragment thereof is generally present in a concentration ranging from about 0.001 % to about 99.9% w/w.

In a preferred aspect, the invention pertains to a pharmaceutical composition in accordance with the invention, for use as a medicament. The invention also relates to the use of such a pharmaceutical composition for the manufacture of a medicament for the treatment of cancer.

In the present context, the term "cancer" refers to a condition, such as breast cancer, prostate cancer, lung cancer, leukaemia, brain tumour, osteosarcoma, skin tumor, liver cancer, kidney cancer, testis cancer, as well as any kind of solid tumours.

In yet another aspect, the invention relates to a method for administering a drug comprising at least one element for association of said drug to said drug delivery system, wherein said method comprises administering a drug delivery system according to the invention, and/or a pharmaceutical composition according to the invention, to a patient in need thereof. In one aspect, said element is a metal element.

### Experimental section

### Expression of an RHCC peptide

The recombinant RHCC polypeptide chain fragment is expressed in *Escherichia coli* strain JM109 (DE3) (Novagen) or some other suitable bacterial strain. A cDNA fragment coding for residues Ile3 to Ile52 of the full-length tetrabrachion molecule and an N-terminal polyhistidine tag is generated by PCR technology using pet15b-RHCC as a template. The PCR product is ligated into the pet15b expression vector (Novagen), or any other suitable expression vector, using appropriate restriction sites. After transformation with the RHCC expression vector bacteria are cultured at 37°C in liquid broth until an optical density of 0.6 at 600nm is reached. The expression of RHCC polypeptide chain fragment is then induced by adding isopropyl beta-D-thiogalactopyranoside (IPTG) at 0.4 mM. The culture is continued for at least 2 hours following induction. The preparation of RHCC peptide from bacterial cells is carried out by harvesting bacteria from the culture medium and performing cell lysis under denaturing conditions. Spontaneous renaturing of RHCC peptide is facilitated by reconstitution of physiological conditions. Purification of the 6xHis-tagged fusion peptide by affinity chromatography on Ni²⁺-Sepharose (Novagen) is performed under denaturing conditions as described in the manufacturer's instructions. Separation of the RHCC polypeptide chain fragment from the 6xHis tag is carried out by protease treatment. The recombinant polypeptide chain fragment contains two additional N-terminal Gly and Ser residues which originate from the expression plasmid and are not part of the tetrabrachion coding sequence.

### Crystallisation

Crystallization experiments are performed at 4 °C employing the vapour diffusion technique. Sitting droplets are made by mixing 4 µl peptide solution (12.8 mg ml⁻¹) with 2 M ammonium sulfate and 200 mM Tris-HCl (pH 7.9). The crystals belong to the space group P3₁21, with dimensions of a=110.02 A, b=110.02 A and c=70.96 A.

### Localisation of a drug

The location of a drug inside the cavities can be detected by techniques sensitive to the local microscopic environment of the molecule, e.g. the drug. For example the absorption of visible light (UV-Vis spectroscopy - solvatochromic effect) can be used to probe the local environment of e.g. Hgl₄⁻ (Figure 1). The position of the absorption peak of Hgl₄⁻ is measured in water (Figure 1 - Plot A), in methanol (Figure 1, Plot C), water/methanol (50/50) (Figure 1 - Plot B) and in a mixture with the peptide (Figure 1 - Plot E). The position of the Hgl₄⁻ peak in the mixture with the peptide coincides with the peak of Hgl₄⁻ in methanol, which indicates that Hgl₄⁻ resides in a hydrophobic environment when mixed with the peptide (compare Figure 1 - plot C with plot E). It can be concluded that the peptide provides a methanol-like hydrophobic environment for the dissolved species (Hgl₄⁻) Hence the Hgl₄⁻ resides inside the hydrophobic cavities when mixed with the peptide in the above fashion.

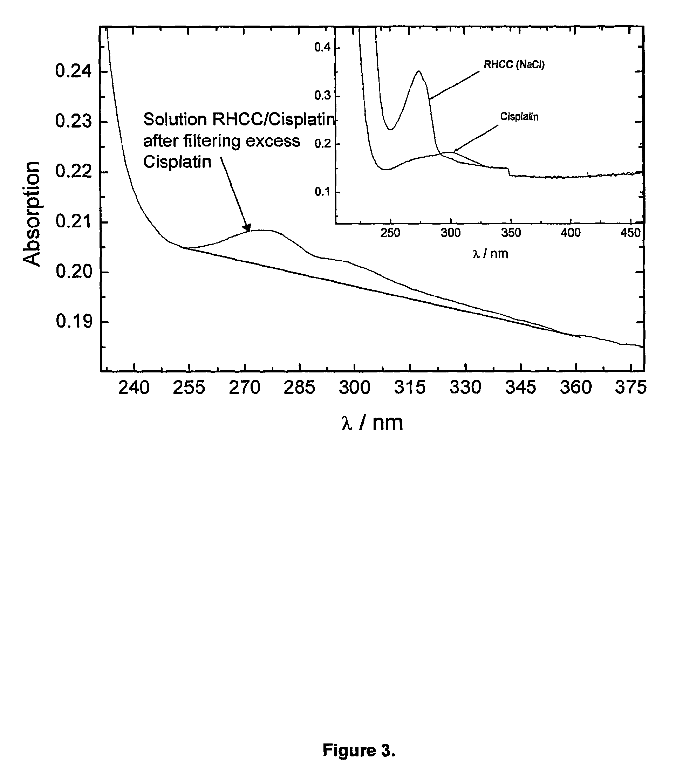

An additional indication of a strong interaction between the metal complexes and RHCC can be established by filtering experiments: Firstly, RHCC is mixed with an excess amount of a metal complex (e.g. Hgl₄²⁻ or cisplatin). After leaving the mixed solution stirred for at least 1 hour, the solvent containing RHCC, the metal complex and the buffer or any other supporting salt, the solvent together with any excess metal complex and salt is replaced via size exclusion filters by a solvent NOT containing any metal complex. The result of such an experiment is plotted in Figure 2. The spectra show that even after separating the excess metal complex from RHCC the typical peaks of Hgl₄²⁻ can be observed. This strongly indicates that the Hgl₄²⁻/RHCC interactions are strong enough to prevent Hgl₄²⁻ from being filtered from the solution. In combination with the x-ray structures a location of Hgl₄²⁻ inside the cavities in solution can be concluded. The same experiment has been performed with cisplatin as shown in Figure 3: The filtered solution of RHCC clearly shows the presence of cisplatin, which indicates a strong interaction between the two components. According to the crystal structure, incorporation into the cavities of RHCC can be concluded for cisplatin.

Other methods to evaluate the incorporation of the charged or uncharged species into the protein is mass-spectrometry (MALDI-TOF), infrared spectroscopy (IR), if the drug has IR active groups, and electron spin resonance (ESR) if the drug has a metal ion with unpaired electrons.

### Fusion molecule of RHCC and Human Interleukin-6

A fusion protein was produced with the following parts in sequence:
i) a histidine tag for purification:
   HHHHHHLVPRGS (SEQ ID NO:2)
ii) a human interleukin-6 sequence:
ii) a GS linker
iii) the RHCC sequence:
   IINETADDIVYRLTVIIDDRYESLKNLITLRADRLEMIINDNVSTILASI (SEQ ID NO:4)

The calculated molecular mass of RHCC-IL-6 monomer is 28.3 kDa. In SDS -PAGE the molecule shows an apparent molecular mass of 32 kDa. The corresponding value for the tetramer is around 130 kDa. The RHCC-IL-6 fusion molecule is soluble in aqueous media and shows full biological activity on the IL-6-dependent BAF/3 cell line. This indicates that the fusion molecule is able to bind to cell surface receptors on living cells and activate them in the manner of the native ligand.

### References

Stetter KO: Life at the upper temperature border. In Colloque Interdisciplinaire du Comité National de la Recherche Scientifique, Frontiers of Life, C55. Edited by Tran Than Van J & K, Mounolou JM, Schneider J, McKay C. Gif-sur-Yvette, France: Editions Frontières; 1993:195-219.
ⁱⁱ J. Stetefeld, M. Jenny, T. Schulthess, R. Landwehr, J. Engel and R. A. Kammerer Nature - Stuct. Biol., 7, (9), 2000, 772 - 776.
ⁱⁱⁱ J. Peters, W. Baumeister and A. Lupas, J. Mol. Biol., 257,1996,1031 -1041.
^{iv} J, Peters, M. Nitsch, B. Kuehlmorgen, R. Golbik, A. Lupas, J. Kellermann, H. Engelhardt, J.-P. Pfander, S. Mueller, K. Goldie, A. Engel, K.-O. Stetter and W. Baumeister, J. Mol. Biol., 245, 1995, 385 - 401.
^{v} J. Mayr, A. Lupas, J. Kellermann, C. Eckerskom, W. Baumeister and J. Peters, Curr. Biol, 6, 1996, 739 - 749.
^{vi} Z. Guo, P.J. Sadler, Angew.Chem.Int.Ed.38, 1999, 1512-1531
^{vii} T. M. Allen, P. R. Cullis, Science, 303, 2004, 1818 -1822.
^{Viii} E. R. Gillies, J. M. J. Frechet, Drug Discovery Today, 10, 2005, 35 - 43.
^{ix} R. Harris, P. O'Shea, Patent WO 02/089852 A1.
^{x} P. Burkhard, Patent WO 2004/071493 A1.
^{xi} Y. B. Yu, Adv. Drug Deliv. Rev., 54, 2002, 1113 - 1129.
^{xii} L. M. Liz-Marzan and P. V. Kamat (Ed.) Nanoscale Materials; Kluwer Academic Publishers: Boston / Dordrecht l London, 2003.
^{xiii} Lupas, A. Trends Biochem. Sci. 21, 375-382 (1996).

## Claims

1. A pharmaceutical composition for use as a medicament, said pharmaceutical composition comprising a drug delivery system, wherein said drug delivery system comprises:
- an RHCC peptide derived from the protein tetrabrachion and
- a drug comprising at least one element selected from the group consisting of Al, Sc, Ti, V, Cr, Fe, Co, Ni, Cu, Zn, Ga, Ge, As, Se, Sr, Y, Zr, Nb, Mo, Tc, Ru, Rh, Pd, Ag, Cd, In, Sn, Sb, Te, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Fr, Ra, Ac, Th, Pa, Np, and Am, and/or a combination thereof.

2. A pharmaceutical composition for use according to claim 1, wherein said drug is a Pt compound.

3. A pharmaceutical composition for use according to claim 1 or 2, wherein said drug is selected from the group of Pt-containing drugs consisting of cisplatin (PtH₆N₂Cl₂), Carboplatin (PtC₆H₁₂N₂O₄), Nedaplatin (PtN₂C₂H₈N₂O₃), Oxaliplatin (PtN₂C₈H₁₄O₄), ZD0473 (PtC₆H₁₀N₂Cₗ₂), JM216 (PtC₁₀H₂₂N₂O₄Cₗ₂), Lobaplatin (PtC₉H₁₈N₂O₃), *trans-*[PtCl₂(pyridine)₂], and *trans* -[Pt(OH)₂Cl₂(NH₃)(NH₂-C₆H₅)], and/or a combination thereof.

4. A pharmaceutical composition for use according to any one of claims 1 to 3, wherein said RHCC peptide further comprises a tag designed to recognise targets on tumour cells.

5. A pharmaceutical composition for use according to claim 4, wherein said tag is an antibody.

6. A pharmaceutical composition for use according to claim 4, wherein said tag is a peptide.

7. A pharmaceutical composition for use according to any one of claims 1 to 6, wherein said RHCC peptide is a fusion peptide.

8. A pharmaceutical composition for use according to claim 7, wherein said RHCC peptide is a fusion peptide comprising human interleukin-6.

9. A pharmaceutical composition according to any one of claims 1 to 8, for use in the treatment of cancer.

10. A pharmaceutical composition for use according to claim 9, wherein said cancer is selected from among breast cancer, prostate cancer, lung cancer, leukaemia, brain tumour, osteosarcoma, skin tumour, liver cancer, kidney cancer, and testis cancer.

## Patentansprüche

1. Pharmazeutische Zusammensetzung zur Verwendung als Arzneimittel, wobei die pharmazeutische Zusammensetzung ein Arzneistoffabgabesystem umfasst, wobei das Arzneistoffabgabesystem Folgendes umfasst:
- ein von dem Protein Tetrabrachion abgeleitetes RHCC-Peptid und
- einen Arzneistoff, der mindestens ein Element, ausgewählt aus der Gruppe bestehend aus Al, Sc, Ti, V, Cr, Fe, Co, Ni, Cu, Zn, Ga, Ge, As, Se, Sr, Y, Zr, Nb, Mo, Tc, Ru, Rh, Pd, Ag, Cd, In, Sn, Sb, Te, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, TI, Pb, Bi, Fr, Ra, Ac, Th, Pa, Np und und/oder einer Kombination davon, umfasst.

2. Pharmazeutische Zusammensetzung zur Verwendung nach Anspruch 1, wobei es sich bei dem Arzneistoff um eine Pt-Verbindung handelt.

3. Pharmazeutische Zusammensetzung zur Verwendung nach Anspruch 1 oder 2, wobei der Arzneistoff aus der Gruppe der Pt-haltigen Arzneistoffe bestehend aus Cisplatin (PtH₆N₂Cl₂), Carboplatin (PtC₆H₁₂N₂O₄), Nedaplatin(PtN₂C₂H₈N₂O₃) Oxaliplatin (PtN₂C₈H₁₄O₄), ZD0473 (PtC₆H₁₀N₂C₁₂) , JM216 (PtC₁₀H₂₂N₂O₄C₁₂), Lobaplatin (PtC₉H₁₈N₂O₃) , trans-[PtCl₂(Pyridin)₂] und *trans*-[Pt(OH)₂Cl₂(NH₃)(NH₃-C₆H₅) und/oder einer Kombination davon ausgewählt ist.

4. Pharmazeutische Zusammensetzung zur Verwendung nach einem der Ansprüche 1 bis 3, wobei das RHCC-Peptid weiterhin einen Tag zum Erkennen von Zielen an Tumorzellen umfasst.

5. Pharmazeutische Zusammensetzung zur Verwendung nach Anspruch 4, wobei es sich bei dem Tag um einen Antikörper handelt.

6. Pharmazeutische Zusammensetzung zur Verwendung nach Anspruch 4, wobei es sich bei dem Tag um ein Peptid handelt.

7. Pharmazeutische Zusammensetzung zur Verwendung nach einem der Ansprüche 1 bis 6, wobei es sich bei dem RHCC-Peptid um ein Fusionspeptid handelt.

8. Pharmazeutische Zusammensetzung zur Verwendung nach Anspruch 7, wobei es sich bei dem RHCC-Peptid um ein Fusionspeptid, das ein Human-Interleukin-6 umfasst, handelt.

9. Pharmazeutische Zusammensetzung nach einem der Ansprüche 1 bis 8 zur Verwendung in der Behandlung von Krebs.

10. Pharmazeutische Zusammensetzung zur Verwendung nach Anspruch 9, wobei der Krebs aus der Reihe Brustkrebs, Prostatakrebs, Lungenkrebs, Leukämie, Hirntumor, Osteosarkom, Hauttumor, Leberkrebs Nierenkrebs und Hodenkrebs ausgewählt ist.

## Revendications

1. Composition pharmaceutique destinée à être utilisée comme médicament, ladite composition pharmaceutique comprenant un système de libération de médicament, dans laquelle ledit système de libération de médicament comprend :
- un peptide RHCC dérivé de la protéine tétrabrachion et
- un médicament comprenant au moins un élément choisi dans le groupe constitué par Al, Sc, Ti, V, Cr, Fe, Co, Ni, Cu, Zn, Ga, Ge, As, Se, Sr Y, Zr, Nb, Mo, Tc, Ru, Rh, Pd, Ag, Cd, In, Sn, Sb, Te, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Fr, Ra, Ac, Th, Pa, Np et Am, et/ou une association de ceux-ci.

2. Composition pharmaceutique destinée à être utilisée selon la revendication 1, dans laquelle ledit médicament est un composé de Pt.

3. Composition pharmaceutique destinée à être utilisée selon la revendication 1 ou 2, dans laquelle ledit médicament est choisi dans le groupe de médicaments contenant du Pt constitué par le cisplatine (PtH₆N₂Cl₂), le carboplatine (PtC₆H₁₂N₂O₄), le nedaplatine (PtN₂C₂H₈N₂O₃), l'oxaliplatine (PtN₂C₈H₁₄O₄),ZD0473 (PtC₆H₁₀N₂Cl₂), JM216 (PtC₁₀H₂₂N₂O₄Cl₂), le lobaplatine (PtC₉H₁₈N₂O₃), le *trans*-[PtCl₂(pyridine)₂] et le trans-[Pt(OH)₂Cl₂(NH₃)(NH₂-C₆H₅)] et/ou une association de ceux-ci.

4. Composition pharmaceutique destinée à être utilisée selon l'une quelconque des revendications 1 à 3, dans laquelle ledit peptide RHCC comprend en outre une étiquette conçue pour reconnaître des cibles sur des cellules tumorales.

5. Composition pharmaceutique destinée à être utilisée selon la revendication 4, dans laquelle ladite étiquette est un anticorps.

6. Composition pharmaceutique destinée à être utilisée selon la revendication 4, dans laquelle ladite étiquette est un peptide.

7. Composition pharmaceutique destinée à être utilisée selon l'une quelconque des revendications 1 à 6, dans laquelle ledit peptide RHCC est un peptide de fusion.

8. Composition pharmaceutique destinée à être utilisée selon la revendication 7, dans laquelle ledit peptide RHCC est un peptide de fusion comprenant l'interleukine-6 humaine.

9. Composition pharmaceutique selon l'une quelconque des revendications 1 à 8, destinée à être utilisée dans le traitement d'un cancer.

10. Composition pharmaceutique destinée à être utilisée selon la revendication 9, ledit cancer étant choisi parmi le cancer du sein, le cancer de la prostate, le cancer du poumon, la leucémie, une tumeur cérébrale, un ostéosarcome, une tumeur cutanée, le cancer du foie, le cancer du rein et le cancer du testicule.


### SEQUENCE LISTING

<110> Juergen Mueller
   Egbert Figgemeier/
   Optovent AB
<120> An RHCC peptide and uses thereof
<130> PD53671PC01
<150> US60/672403
   <151> 2005-04-18
<160> 4
<170> FastSEQ for Windows Version 4.0
<210> 1
   <211> 52
   <212> PRT
   <213> Artificial Sequence
<220>
   <223> RHCC peptide including GS linker in N-terminal
<400> 1
   Gly Ser Ile Ile Asn Glu Thr Ala Asp Asp Ile Val Tyr Arg Leu Thr
    1               5                   10                  15
   Val Ile Ile Asp Asp Arg Tyr Glu Ser Leu Lys Asn Leu Ile Thr Leu
               20                  25                  30
   Arg Ala Asp Arg Leu Glu Met Ile Ile Asn Asp Asn Val Ser Thr Ile
           35                  40                  45
   Leu Ala Ser Ile
       50
<210> 2
   <211> 12
   <212> PRT
   <213> Artificial Sequence
<220>
   <223> Histidine tag
<400> 2
   His His His His His His Leu Val Pro Arg Gly Ser
    1               5                   10
<210> 3
   <211> 185
   <212> PRT
   <213> Human
<400> 3
   Met Pro Val Pro Pro Gly Glu Asp Ser Lys Asp Val Ala Ala Pro His
    1               5                  10                  15
   Arg Gln Pro Leu Thr Ser Ser Glu Arg Ile Asp Lys Gln Ile Arg Tyr
               20                  25                  30
   Ile Leu Asp Gly Ile Ser Ala Leu Arg Lys Glu Thr Cys Asn Lys Ser
           35                  40                  45
   Asn Met Cys Glu Ser Ser Lys Glu Ala Leu Ala Glu Asn Asn Leu Asn
       50                  55                  60
   Leu Pro Lys Met Ala Glu Lys Asp Gly Cys Phe Gln Ser Gly Phe Asn
   65                  70                  75                      80
   Glu Glu Thr Cys Leu Val Lys Ile Ile Thr Gly Leu Leu Glu Phe Glu
                   85                  90                  95
   Val Tyr Leu Glu Tyr Leu Gln Asn Arg Phe Glu Ser Ser Glu Glu Gln
               100                 105                 110
   Ala Arg Ala Val Gln Met Ser Thr Lys Val Leu Ile Gln Phe Leu Gln
           115                 120                 125
   Lys Lys Ala Lys Asn Leu Asp Ala Ile Thr Thr Pro Asp Pro Thr Thr
       130                 135                 140
   Asn Ala Ser Leu Leu Thr Lys Leu Gln Ala Gln Asn Gln Trp Leu Gln
   145                 150                 155                     160
   Asp Met Thr Thr His Leu Ile Leu Arg Ser Phe Lys Glu Phe Leu Gln
                   165                 170                 175
   Ser Ser Leu Arg Ala Leu Arg Gln Met
               180                 185
<210> 4
   <211> 50
   <212> PRT
   <213> Staphylothermus marinus
<400> 4
   Ile Ile Asn Glu Thr Ala Asp Asp Ile Val Tyr Arg Leu Thr Val Ile
    1               5                  10                  15
   Ile Asp Asp Arg Tyr Glu Ser Leu Lys Asn Leu Ile Thr Leu Arg Ala
               20                  25                  30
   Asp Arg Leu Glu Met Ile Ile Asn Asp Asn Val Ser Thr Ile Leu Ala
           35                  40                  45
   Ser Ile
       50